# EUROPEAN PATENT SPECIFICATION

(11) **EP 1 409 070 B1**
(45) Date of publication and mention of the grant of the patent: **22.12.2010**
(21) Application number: 02739599.5
(22) Date of filing: 31.05.2002
(51) Int. Cl.: A61N 1/375, H01R 13/52, H01R 4/48, A61N 1/372, A61N 1/05

(54) **MINIATURE IMPLANTABLE CONNECTORS**
IMPLANTIERBARE MINIATURVERBINDER
CONNECTEURS MINIATURES IMLPANTABLES

(30) Priority: 18.06.2001 US 299106 P; 04.10.2001 US 971848; 04.10.2001 US 971849
(43) Date of publication of application: 21.04.2004
(60) Divisional application: 04002652.8
(73) Proprietor: Alfred E. Mann Foundation for Scientific Research, Santa Clarita, CA 91380-9005 (US)
(72) Inventor: SCHULMAN, Joseph, H., Santa Clarita, CA 91351 (US); FEY, Kate, E., Valencia, CA 91355 (US); BYERS, Charles, L., Canyon Country, CA 91351 (US); ZILBERMAN, Yitzhak, Santa Clarita, CA 91321 (US); DELL, Robert, D., Valencia, CA 91354 (US); KUZMA, Janusz, Parker, CO 80138 (US)
(74) Representative: Paget, Hugh Charles Edward
(86) International application number: PCT/US2002/017353
(87) International publication number: WO 2002/102452

(56) References cited:
- WO-A-03/063951
- CA-A1- 2 171 067
- US-A- 3 340 493
- US-A- 5 193 539
- US-A- 5 358 514
- US-A- 5 906 634
- US-A- 6 051 017
- US-A1- 2003 114 905
- US-B1- 6 185 452

## Description

### FIELD OF THE INVENTION

This invention relates to a prosthetic medical device and methods, and more particularly to methods of connecting electrical conducting wires to a miniature implantable device to minimize risk to the living tissue during and after surgery,

### BACKGROUND OF THE INVENTION

Neurological disorders are often caused by neural impulses failing to reach their natural destination in otherwise functional body systems. Local nerves and muscles may function, but, for various reasons, such as injury, stroke, or other cause, the stimulating nerve signals do not reach their natural destination. For example, paraplegic and quadriplegic animals have intact nerves connected to functioning muscles and only lack the brain-to-nerve link. Electrically stimulating the nerve or muscle can provide a useful muscle contraction.

Further, implanted devices may be sensors as well as stimulators. In either case, difficulties arise both in providing suitable, operable stimulators or sensors which are small in size and in passing sufficient energy and control information to or from the device, with or without direct connection, to satisfactorily operate them.

In CA 2171067, the author describes "a method of controlling human nerve activity in a human body, the method comprising the step of applying electrical pulses to a neuron of a human nerve", and Schulman, et al discuss that "an addressable, Implantable microstimulator is substantially encapsulated within a hermetically-sealed housing inert to body fluids, and of a size and shape capable of implantation in a living body" in US-A-5193539. Miniature monitoring and/or stimulating devices for implantation in a living body are further disclosed by Schulman, et al in U.S. Patent numbers 6164284, 6185452 and 6208894.

It must be assured that the electrical current flow does not damage the intermediate body cells or cause undesired stimulation. Anodic or cathodic deterioration of the stimulating electrodes must not occur.

In addition, at least one small stimulator or sensor disposed at various locations within the body may send or receive signals via electrical wires. The implanted unit must be sealed to protect the internal components from the body's aggressive environment, If wires are attached to the stimulator, then these wires and the area of attachment must be electrically insulated to prevent undesired electric signals from passing to surrounding tissue.

Miniature stimulators offer the benefit of being locatable at a site within the body where a larger stimulator cannot be placed because of its size. The miniature stimulator may be placed into the body by injection. The miniature stimulator offers other improvements over larger simulator in that they may be placed in the body with little or no negative cosmetic effect. There may be locations where these miniature devices do not fit for which it is desired to send or receive signals. Such locations include, but are not limited to, the tip of a finger for detection of a stimulating signal or near an eyelid for stimulating blinking. In such locations, the stimulator and its associated electronics are preferably located at a distance removed from the sensing or stimulating site within the body; thus creating the need to carry electrical signals from the detection or stimulation site to the remote miniature stimulator, where the signal wire must be securely fastened to the stimulator.

Further, the miniature stimulator may contain a power supply that requires periodic charging or require replacement, such as a battery. When this is the case, the actual stimulation or detection site may be located remotely from the stimulator and may be located within the body, but removed a significant distance from the skin surface. By having the ability to locate the miniature stimulator near the skin while the stimulation site is at some distance removed from the skin, the miniature stimulator and its associated electronics can be more effectively replaced by a surgical technique or more efficiently recharged through the skin by any of several known techniques, including the use of alternating magnetic fields. If the electronics package is replaced surgically, then it is highly desirable to have the capability to reconnect the lead wires to the miniature stimulator via an easy, rapid and reliable method, as disclosed herein.

### SUMMARY OF THE INVENTION

The instant invention provides a structure as set forth in claim 1, in which an electrically conductive wire is connected to a miniature, implantable device. The device case is comprised of electrically insulating material such as plastic or ceramic. The plastic may be epoxy, polycarbonate, or plexiglass. The ceramic may be alumina, glass, titania, zirconia, stabilized-zirconia, partially-stabilized zirconia, tetragonal zirconia, magnesia-stabilized zirconia, ceria-stabilized zirconia, yttria-stabilized zirconia, or calcia-stabilized zirconia. There is at least one electrically conductive electrode for conducting electrical signals. The materials comprising such electrically conductive parts are selected to reduce or eliminate damage due to corrosion from the tissue environment surrounding the miniature stimulator, and also to avoid damage to the tissue, for example, not being toxic or having sharp corners that can damage the tissue.

The electrical connection between the electrically conductive case parts and the electrically conductive wires is accomplished by a spring clip.

The electrode may be either a male pin or a female receptor configuration. Apparatuses for insulating the electrode from the body and for making attachment of a wire to the electrode are disclosed.

In any of these approaches to making a secure and safe connection of wire to connector attachment, the entire connection area and wire must be electrically insulated from the body. Placing a flexible insulating boot over the entire stimulation wire connection accomplishes this. The insulating boot is preferably held in place with at least one of several methods, including ties, C-clips, silicone adhesive or a tight fit with or without a securement ridge.

Each connection mechanism allows for the use of a wire with at least one separate element, each of which may carry an independent electrical signal.

This invention offers a variety of configurations to the surgeon, both pre-surgery and during surgery. Changes may be made to the configuration to accommodate necessary modifications during surgery and during secondary surgeries at a later time. Corrosion is prevented or significantly reduced by the proper selection of materials and the use of an electrically insulating boot in combination with secure attachment methods.

### OBJECTS OF THE INVENTION

It is an object of the invention to provide an implantable miniature stimulator having at least one electrode.

It is an object of the invention to provide a method of connecting at least one wire to a miniature stimulator in a body.

It is an object of the invention to increase the ease and safety of a surgeon making electrical connections for *in vivo* application of a miniature implantable stimulator.

It is an object of the invention to connect the electrode of a miniature implantable stimulator in a secure, safe and rapid fashion to electrical wires.

It is an object of the invention to electrically insulate the electrode of an implantable miniature stimulator that is connected to an electrical wire from the body environment in which it is implanted.

Other objects, advantages and novel features of the present invention will become apparent from the following detailed description of the invention when considered in conjunction with the accompanying drawings.

### BRIEF DESCRIPTION OF THE DRAWINGS

**FIG**. **1** illustrates a perspective view of the miniature stimulator with a threaded connector and nut, which is outside the scope of the invention.
**FIG**. **2** illustrates a perspective view of the smooth nut with a flare nut cap, which is outside the scope of the invention.
**FIG**. **3** is a cross-section through the flare nut wire insertion.
**FIG**. **4** is a cross-sectional view of the smooth cap with flare insertion.
**FIG**. **5** is a longitudinal section showing the flare nut, which is outside the scope of the invention, with a rubber boot which is part of the invention.
**FIG**. **5A** is a section showing tie interaction with the rubber boot of **FIG**. **5**.
**FIG. 6** illustrates a perspective view of an embodiment of the invention, an electrically conductive doorknob shaped electrode with spring clip connector and wire.
**FIG. 7** is a perspective view of the electrically conductive doorknob shaped electrode.
**FIG. 8** is a perspective view of the spring clip connector.
**FIG**. **9** is a longitudinal section through the doorknob shaped connector with a wire and rubber boot.
**FIG**. **10** is a longitudinal section through the doorknob shaped connector with crimped connector a wire and rubber boot.
**FIG**. **11** is a hand showing placement of an implantable miniature device with a wire lead that carries electrical signals to a fingertip.

### DETAILED DESCRIPTION OF THE PREFERRED EMBODIMENT

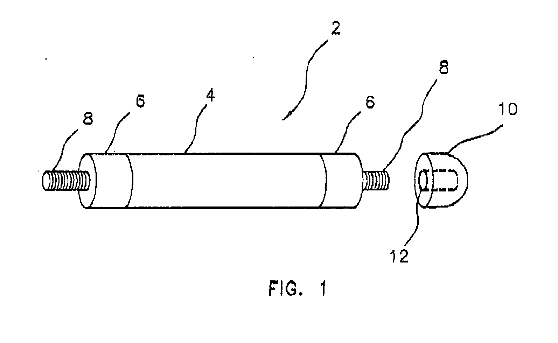

An example of an implantable miniature stimulator 2 is illustrated in **FIG. 1**. **FIG**. **11** represents a typical placement of the implantable miniature stimulator 2 at a location that is remote from the site that is to be stimulated, in this case a fingertip, where an electrically conductive wire 38 carries the electrical signal to an electrode 39 at the stimulation site. Typical dimensions for this device are about 5 to 60 mm in length and about 1 to 6 mm in diameter. (See, for example, U.S. Patent Nos. 6,164,284, 6,185,452, and 6,208,894 which are Incorporated herein by reference in their entirety.) While element 2 is generally described as a stimulator, it is recognized that the present invention is equally applicable when element 2 is operable as a sensor or as a stimulator and a sensor, Stimulator 2 includes insulating case 4, which typically is hollow and contains an electronics package and a power source, such as a battery, capacitor, magnetic field to electricity converter, and electrically conductive case ends 6, each of which has an electrically conductive electrode 8 which conducts electrical signals from a stimulator and/or to a sensor, depending upon the design and function of that particular miniature stimulator 2. Stimulator 2 may have at least one electrode, e.g., 2-8 or more, depending upon its particular design and function, although, for illustrative purposes, only two electrodes are shown in **FIG. 1**. Electrically conductive electrodes 8 are shown threaded in **FIG. 1**, although those of the claimed invention, and other disclosures, are shown in other figures and are discussed herein.

Insulating case 4 contains the electronics, which may include a battery or other energy storage device and signal generating or receiving circuitry and is made of an electrically insulating material that is capable of being hermetically sealed and that is also biocompatible, such as plastic or ceramic. The plastic may be epoxy, polycarbonate, or plexiglass. The ceramic may be alumina, glass, titania, zirconia, stabilized-zirconia, partially-stabilized zirconia, tetragonal zirconia, magnesia-stabilized zirconia, ceria-stabilized zirconia, yttria-stabilized zirconia, or calcia-stabilized zirconia, and in a preferred embodiment, insulating case 4 is yttria-stabilized zirconia, although other insulating materials may also be used. The insulating case 4 must be a material that is biocompatible as well as capable of being hermetically sealed, to prevent permeation of bodily fluids into the case.

The electrically conductive case end 6 is preferably a biocompatible, non-corrosive material, such as titanium or a titanium alloy, although other metals such as platinum, iridium, platinum-iridium, stainless steel, tantalum, niobium, or zirconium may be used. The preferred material Is Ti- 6 Al- 4 V. An alternate preferred material is platinum-iridium.

If any electrically conductive electrode is not being used while the stimulator is in the body, then the electrode may be insulated to prevent stimulation of nearby tissue. Protective nut 10 is either an insulator or an electrically conductive conductor. If it is an electrical conductor, then it is an extension electrode of electrically conductive case 6. It is placed over the unused electrically conductive electrode 8 such that in this example of threaded electrodes, which is outside the scope of the invention, protective nut threaded hole 12 is tightly screwed onto threaded electrically conductive electrode 8. In a preferred example of this disclosure, the threads on threaded electrically conductive electrode 8 are 0-80 threads. In order to avoid growth of tissue into joints, such as the joint formed between protective nut 10 and electrically conductive case end 6, it is preferable that any gap be less than 7 microns.

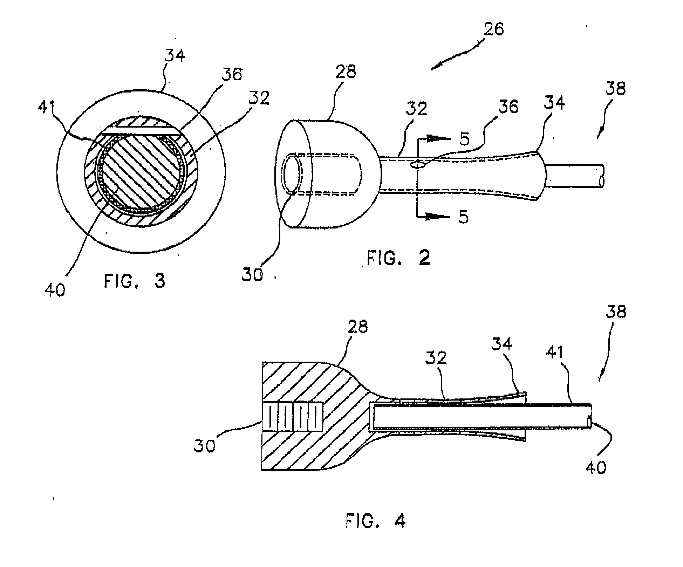

A further example of a method of attaching an electrically conductive wire 38 to a miniature stimulator 2 (see **FIG. 1**), which falls outside the scope of the invention is illustrated in **FIGS. 2, 3** and **4** wherein flare nut 26 is comprised of protective nut 28, which contains flare nut mounting hole 30. Threaded flare nut mounting hole 30 is positioned over electrode 8 (see FIG. 1) and tightened by screwing onto the threads. Flare nut 26 also contains flare nut wire receptor 32 which has flare 34 on its extension pointed away from protective nut 28. Because of the small diameter of wire used in this application, flare 34 is provided for ease of placement of electrically conductive wire 38 into flare 34. Offset through-hole 36 passes through flare nut wire receptor 32 in a plane that is perpendicular to the longitudinal axis of flare nut 26. Offset through-hole 36 preferably does not intersect with the longitudinal axis of nut 26 but is intentionally offset to penetrate wire insulator 41 (see **FIG**. **4**) and to intersect with the outer diameter of wire conductor 40. Thus when a pin, not illustrated, is placed in offset through-hole 36, wire conductor 40 is contacted, creating an electrically conductive path between wire conductor 40 and protective nut 28.

The cross-sectional view of **FIG**. **3** illustrates the offset alignment of offset through-hole 36 with respect to the longitudinal axis of flare nut wire receptor 32. Wire conductor 40 is intersected by offset through-hole 36 such that wire insulator 41 will be penetrated and wire conductor 40 will be contacted by a pin inserted in offset through-hole 36. Electrically conductive wire 38, shown in **FIG**. **4** is comprised of wire conductor 40 within wire insulator 41. Alternately, wire insulator 41 may be stripped from an end portion of wire conductor 40, to help insure good electrical contact between conductor 40 and flare nut wire receptor 32.

In a preferred example of this disclosure, wire conductor 40 is a highly conductive metal that is also benign in the body, such as MP35, although stainless steel or an alloy of platinum-iridium may also be used. Preferably, the wire has a diameter of approximately 0.003 inches. It is contained in wire insulator 41 to electrically isolate it from the body tissue and fluids and, In a preferred embodiment, wire insulator 41 is Teflon-coated silicone.

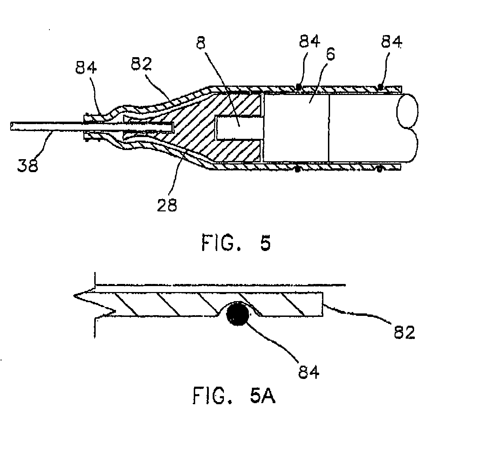

The preferred method of assuring electrical insulation between electrically conductive case end 6, electrically conductive electrode 8, protective nut 28, and wire 38, as illustrated in **FIG. 5** and forming part of the invention, is to cover the electrically conductive case end 6 and other parts with rubber boot 82. Rubber boot 82 is made of a flexible insulating material that is biocompatible, such as silicone. Its purpose is to provide electrical insulation such that stray electrical signals do not pass between surrounding tissue and any electrically conductive part of the device. Rubber boot 82 is secured to the device, preferably by tying it in place with ties 84. A sufficient number of ties 84 are placed by the surgeon to assure that that the rubber boot 82 will not move. It is preferred that at least one tie 84 and, preferably two or more ties 84, be placed on rubber boot 82 to secure rubber boot 82 to insulating case 4, so as to electrically insulate electrically conductive case end 6 from the living tissue. **FIG**. **5A** illustrates a typical tie 84 interacting with rubber boot 82, so as to establish and maintain a hermetic seal. Alternate methods of attaching rubber boot 82 include the use of ridges inside rubber boot 82, clamps over rubber boot 82, silicone adhesive inside rubber boot 82, ridges on the outside of insulating case 4, a male notch with matching female indentation forming an O-ring seal, and the tight fit of rubber boot 82 over the device, either with or without internal ridges.

An embodiment of the invention for making an electrical connection to a miniature stimulator 2 is illustrated in **FIG**. **6** where doorknob electrode 108 is intimately attached to electrically conductive case end 6. The doorknob electrode is made of a material that is electrically conductive and biocompatible, such as titanium. Spring clip 110 is preferably a clip made of titanium which has two or more, and preferably three or four prongs. Wire insulator 41 is stripped from the end of wire 38 thereby exposing wire conductor 40. Wire conductor 40 is preferably attached to spring clip 110 by strain relief weld 112. Strain relief weld 112 helps to relieve strain in wire conductor 40 by virtue of being oriented perpendicular to the longitudinal axis of miniature stimulator 2. Further strain relief is provided in wire conductor 40 by virtue of it being tightly coiled inside wire insulator 41 thereby forming wire strain relief 114. The inside of wire insulator 41 is fill material 115, which is preferably soft silicone, to minimize infiltration of body fluids and other tissue inside wire 38.

A perspective view of doorknob electrode 108, showing its end attached to electrically conductive case end 6, is illustrated in **FIG**. 7. **FIG**. **8** illustrates a perspective view of spring clip 110 showing the four prongs that slip over doorknob electrode 108 to form an electrical connection.

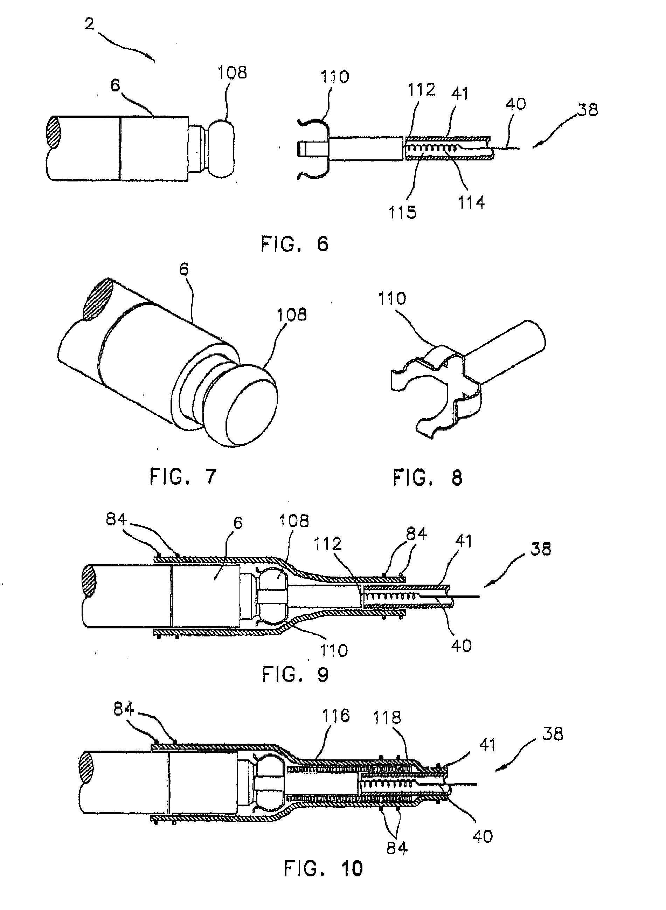

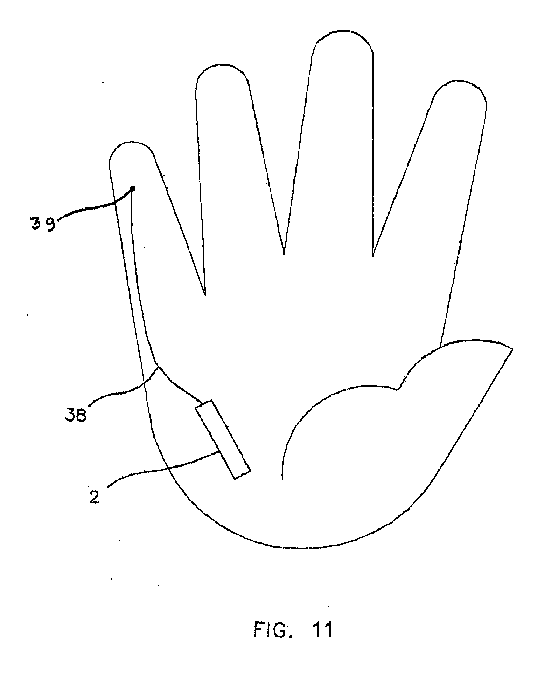

**FIG. 9** illustrates spring clip 110 together with electrically conductive wire 38, which in turn is attached by strain relief weld 112 to wire conductor 40. Spring clip 110 is shown in its attached position on doorknob electrode 108. Rubber boot 82 is securely fastened to the device with ties 84 to completely cover electrically conductive case end 6, doorknob electrode 108, wire conductor 40 and a portion of wire insulator 41, thus electrically insulating the body tissue from electrical signals.

An alternate embodiment is presented in **FIG**. **10**, which is similar to the connection device presented in **FIG**. **9** except that connector crimp 118, which is selected from the group of biocompatible materials, and is preferably platinum metal, is placed over the end of electrically conductive wire 38 so as to cover a portion of wire insulator 41 and stripped wire conductor 40. Connector crimp 118 is attached to electrically conductive wire 38 by crimping it onto wire 38.

These various embodiments of the invention, and examples of similar technology, are of devices and methods for connecting an electrically conductive wire to a miniature, implantable stimulator in order to efficiently transmit or receive an electrical signal that is associated with the implantable stimulator.

Obviously, these methods of attaching a wire to a miniature implantable stimulator can be used in permutations and combinations not specifically discussed herein. Many modifications and variations of the present invention are possible in light of the above teachings, It is therefore to be understood that, within the scope of the appended claims, the invention may be practiced otherwise than as specifically described.

## Claims

1. A structure comprising:
- an implantable miniature device (2) configured for monitoring and/or affecting body parameters, wherein said implantable miniature device (2) has an axial dimension of less than about 60 mm and a lateral dimension of less than about 6 mm, an insulating case (4) and at least one electrically conductive case end (6) coupled to electrical circuitry contained within the insulating case (4), said at least one electrically conductive case end comprising at least one doorknob electrode (108) for communicating electrical signals between living tissue and said implantable miniature device,
- at least one electrically conductive wire (40) having a first end configured for electrical coupling to a selected portion of the living tissue and a second end configured for coupling to said implantable miniature device;
**characterised in that** the structure further comprises:
- a spring clip (110) for attaching said at least one electrically conductive wire (40) to said at least one doorknob electrode (108); and
- an insulating rubber boot (82) surrounding said at least one electrically conductive case end, said at least one doorknob electrode, and said at least one electrically conductive wire.

2. The structure of claim 1 wherein said at least one electrically conductive case end (6) is a material selected from the group consisting of titanium, titanium alloy, platinum, iridium, platinum-iridium, zirconium, niobium, stainless steel, and tantalum.

3. The structure of claim 1 wherein said at least one electrically conductive case end (6) is comprised of Ti-6AI-4V.

4. The structure of claim 1 wherein said at least one doorknob electrode (108) is a material selected from the group consisting of titanium, titanium alloy, platinum, iridium, platinum-iridium, stainless steel, tantalum and niobium.

5. The structure of claim 1 wherein said insulating rubber boot (82) is comprised of silicone.

6. A spring clip connector (110) adapted to receive a doorknob electrode (108) for communicating electrical signals between living tissue and an implantable miniature device (2) configured for monitoring and/or affecting body parameters, comprising:
at least one prong for grasping said doorknob electrode (108),
a connection to at least one electrically conductive wire (40) having a first end configured for electrical coupling to a selected portion of the living tissue and a second end configured for attachment to said spring clip, and
wherein said spring clip is a biocompatible material.

## Patentansprüche

1. Struktur, die Folgendes umfasst:
- eine implantierbare Miniaturvorrichtung (2), die ausgebildet ist, um Körperparameter zu überwachen und/oder zu beeinflussen, wobei die implantierbare Miniaturvorrichtung (2) eine axiale Abmessung von weniger als etwa 60 mm und eine Seitenabmessung von weniger als etwa 6 mm aufweist, ein Isolationsgehäuse (4) und zumindest ein elektrisch leitfähiges Gehäuseende (6), das mit einer in dem Isolationsgehäuse (4) enthaltenen elektrischen Schaltung verbunden ist, wobei das zumindest eine elektrisch leitfähige Gehäuseende zumindest eine Türknopfelektrode (108) umfasst, um elektrische Signale zwischen lebendem Gewebe und der implantierbaren Miniaturvorrichtung zu übertragen,
- zumindest einen elektrisch leitfähigen Draht (40) mit einem ersten Ende, das ausgebildet ist, um mit einem ausgewählten Abschnitt des lebenden Gewebes elektrisch verbunden zu werden, und einem zweiten Ende, das ausgebildet ist, um mit der implantierbaren Miniaturvorrichtung verbunden zu werden; **dadurch gekennzeichnet, dass** die Struktur ferner Folgendes umfasst:
- eine Federklammer (110) zur Befestigung des zumindest einen elektrisch leitfähigen Drahts (40) an der zumindest einen Türknopfelektrode (108) und
- eine Isolationsgummimanschette (82), die das zumindest eine elektrisch leitfähige Gehäuseende, die zumindest eine Türknopfelektrode und den zumindest einen elektrisch leitfähigen Draht umgibt.

2. Struktur nach Anspruch 1, worin das zumindest eine elektrisch leitfähige Gehäuseende (6) aus einem aus der aus Titan, Titanlegierungen, Platin, Iridium, Platin-Iridium, Zirconium, Niobium, Edelstahl und Tantal bestehenden Gruppe ausgewählten Material besteht.

3. Struktur nach Anspruch 1, worin das zumindest eine elektrisch leitfähige Gehäuseende (3) aus Ti-6Al-4V besteht.

4. Struktur nach Anspruch 1, worin die zumindest eine Türknopfelektrode (108) aus einem aus der aus Titan, Titanlegierungen, Platin, Iridium, Platin-Iridium, Zirconium, Niobium, Edelstahl und Tantal bestehenden Gruppe ausgewählten Material besteht.

5. Struktur nach Anspruch 1, worin die Isolationsgummimanschette (82) aus Silikon besteht.

6. Federklammerverbindungselement (110), das geeignet ist, um eine Türknopfelektrode (108) zur Übertragung von elektrischen Signalen zwischen lebendem Gewebe und einer implantierbaren Miniaturvorrichtung (2) aufzunehmen, die ausgebildet ist, um Körperparameter zu überwachen und/oder zu beeinflussen, wobei das Federklammerverbindungselement Folgendes umfasst:
zumindest eine Spitze zum Fassen der Türknopfelektrode (108),
eine Verbindung zu zumindest einem elektrisch leitfähigen Draht (40) mit einem ersten Ende, das ausgebildet ist, um mit einem ausgewählten Abschnitt des lebenden Gewebes elektrisch verbunden zu werden, und einem zweiten Ende, das ausgebildet ist, um an der Federklammer befestigt zu werden,
wobei die Federklammer aus einem bioverträglichen Material besteht.

## Revendications

1. Structure comprenant:
- un dispositif miniature implantable (2) configuré pour surveiller et/ou agir sur des paramètres corporels, où ledit dispositif miniature implantable (2) a une dimension axiale inférieure à environ 60 mm, et une dimension latérale inférieure à environ 6 mm, un boîtier d'isolation (4) et au moins une extrémité de boîtier électriquement conductrice (6) couplée à des circuits électriques se trouvant dans le boîtier d'isolation (4), ladite au moins une extrémité de boîtier électriquement conductrice comprenant au moins une électrode en bouton de porte (108) pour communiquer des signaux électriques entre le tissu vivant et ledit dispositif miniature implantable,
- au moins un fil électriquement conducteur (40) ayant une première extrémité configurée pour le couplage électrique à une portion sélectionnée du tissu vivant et une seconde extrémité configurée pour le couplage audit dispositif miniature implantable;
**caractérisée en ce que** la structure comprend en outre:
une attache à ressort (110) pour fixer ledit au moins un fil électriquement conducteur (40) à ladite au moins une électrode en bouton de porte (108); et
une botte isolante en caoutchouc (82) entourant ladite au moins une extrémité de boîtier électriquement conductrice, ladite au moins une électrode en bouton de porte et ledit au moins un fil électriquement conducteur.

2. Structure selon la revendication 1, dans laquelle ladite au moins une extrémité de boîtier électriquement conductrice (6) est un matériau sélectionné dans le groupe consistant en titane, alliage de titane, platine, iridium, platine-iridium, zirconium, niobium, acier inoxydable et tantale.

3. Structure selon la revendication 1, dans laquelle ladite au moins une extrémité de boîtier électriquement conductrice (6) est réalisée en Ti-6Al-4V.

4. Structure selon la revendication 1, dans laquelle ladite au moins une électrode en bouton de porte (108) est un matériau sélectionné dans le groupe consistant en titane, alliage de titane, platine, iridium, platine-iridium, acier inoxydable, tantale et niobium.

5. Structure selon la revendication 1, dans laquelle ladite botte isolante en caoutchouc (82) est réalisée en silicone.

6. Connecteur d'attache à ressort (10) apte à recevoir une électrode en bouton de porte (108) pour la communication de signaux électriques entre du tissu vivant et un dispositif miniature implantable (2), configuré pour surveiller et/ou agir sur des paramètres corporels, comprenant:
au moins une griffe pour saisir ladite électrode en bouton de porte (108),
une connection à au moins un fil électriquement conducteur (40) ayant une première extrémité configurée pour le couplage électrique à une portion sélectionnée du tissu vivant et une seconde extrémité configurée pour la fixation à ladite attache à ressort, et
où ladite attache à ressort est un matériau biocompatible.
